Europäisches Patentamt

European Patent Office

Office européen des brevets

(11) Publication number: **0 106 026**
**B2**

(12) **NEW EUROPEAN PATENT SPECIFICATION**

(45) Date of publication of the new patent
specification: **13.06.90**

(21) Application number: **83106507.3**

(22) Date of filing: **04.07.83**

(51) Int. Cl.⁵: **A 61 M 1/14**

(54) A tube set intended for extracorporeal treatment of blood and similar perishable liquids.

(30) Priority: **10.09.82 SE 8205160**

(43) Date of publication of application:
**25.04.84 Bulletin 84/17**

(45) Publication of the grant of the patent:
**29.07.87 Bulletin 87/31**

(45) Mention of the opposition decision:
**13.06.90 Bulletin 90/24**

(84) Designated Contracting States:
**AT BE CH DE FR GB IT LI LU NL**

(56) References cited:
**EP-A-0 058 325**
**EP-A-0 082 721**
**WO-A-81/01793**
**FR-A-2 085 299**
**GB-A-1 560 660**
**GB-A-2 021 418**
**GB-A-2 028 976**
**GB-A-2 063 108**
**US-A-3 834 386**
**US-A-4 102 655**
**US-A-4 681 606**

(73) Proprietor: **Gambro Lundia AB**
**Box 10101**
**S-220 10 Lund (SE)**

(72) Inventor: **Carlsson, Per-Olov Arne Vilhelm**
**Morkullevägen 11**
**S-280 10 Sösdala (SE)**
Inventor: **Karlberg, Rolf Emil**
**Ympningsvägen 32**
**S-240 10 Veberöd (SE)**

(74) Representative: **Boberg, Nils Gunnar Erik**
**Gambro AB Patent Department Box 10101**
**S-220 10 Lund (SE)**

EP 0 106 026 B2

Courier Press, Leamington Spa, England.

## Description

### Technical Field

The present invention relates to a tube set intended for extracorporeal treatment of blood and similar perishable liquids, e.g. in conjunction with dialysis, comprising at least one drip chamber with an inlet and an outlet. Such tube sets normally are made to be used only once. They are frequently divided into an arterial part and a venous part, the arterial part being intended to be coupled between one of the arteries of the patient and the apparatus by means of which the blood is to be treated. The venous part is intended instead to be coupled between the said apparatus and one of the veins of the patient for the return of the blood. The expression tube set in the foregoing and in the following refers first and foremost to the venous part. However, the invention may also be applied to an arterial part or to a tube set consisting of combinations of an arterial part and a venous part.

### Background Art

The venous part of the above discussed tube set includes normally a drip chamber having its inlet and outlet vertically arranged. Drip chambers of that kind are shown in for instance US—A—3 834 386 and FR—A—2 085 299. By such drip chambers there is a great risk for kinking of the tubes and especially of the tube leading to the upper inlet. Said risk may however be reduced if the inlet and the outlet are arranged at an angle in relation to one another as shown in for instance the GB—A—1 560 660. Independent of how the inlet and the outlet are arranged there is a problem in that samples are taken after the dripping. One object of the present invention is therefore to solve as well this problem as the problem of the above-mentioned kinking of the tubes.

Present tube sets normally consist of a number of tubes which are joined to one another by a number of branch pipes, T-pipes or other components such as pump segments, drip chambers, pressure monitoring pads or the like by means of which the flowing medium can be controlled and/or guided. At the same time the tube set normally includes different types of ports for the feed and/or withdrawal of liquid, e.g. for dilution and/or sampling. The said component are normally distributed at different points along the tube set.

The present invention has therefore the further object of facilitating the assembling of the tube set on the front of a control unit, the above-mentioned components and functions associated therewith being concentrated on one or a small number of points in particular on the front of this control unit. If possible, therefore, the tube set should consist of an unbroken tube between the front of this control unit and the patient. In this manner the personnel performing the treatment can concentrate on the control unit and on the patient. The invention is also intended to facilitate sampling and other treatment of the patient.

### Disclosure of Invention

The invention thus relates to a tube set intended for extracorporeal treatment of blood and similar perishable liquids, e.g. in conjunction with dialysis, comprising at least one drip chamber with an inlet and an outlet arranged at an angle in relation to one another. The invention is characterized by a sampling port arranged in the drip chamber in connection with the inlet, upstream of a point where a duct extending from the inlet is directed downwards into the drip chamber. Thus the sampling can be carried out before the drip and with the help of a short suction needle. In this manner the danger of the tubes connected to the drip chamber being constricted as a result of kinking is also reduced. Furthermore new possiblities for the placing of ports for the feed or withdrawal to or from the flowing medium e.g. for sampling and/or dilution are procured.

The drip chamber preferably is given an elongate shape with the outlet at its lower end and arranged in its longitudinal direction. The inlet may be arranged from the side in a lidlike part.

Moreover, the drip chamber may comprise one or more ports for the withdrawal and/or feed of a material, e.g. a liquid or air. The feed or withdrawal of air will be relevant in conjunction with level control in the drip chamber. This level control may be done in a known manner with the help of a simpley syringe. One or more of the said ports are appropriately arranged from the side in a lidlike part.

A preferred embodiment of the object of the invention is intended to constitute a venous set in dialysis or similar treatment and is characterized in that all functions have been concentrated on the drip chamber, as a result of which the tube parts included are completely unbroken between the dialyser or corresponding treatment arrangement and the drip chamber as well as between the latter and the patient. In this way the personnel performing the treatment can concentrate on the control unit, to which the drip chamber is normally attached and on the patient.

### Brief Description of Drawings

Fig. 1 shows schematically a dialyser together with a control unit intended for the same and a tube set joining together these components and the patient, consisting of an arterial part and a venous part.

Fig. 2 and 3 shows two views at right angles to one another of a lidlike part intended to constitute the top part of the drip chamber included in the tube set according to Fig. 1.

Fig. 4, 5 and 6 show sections along the lines IV—IV, V—V and VI—VI respectively.

### Best Mode for Carrying Out the Invention

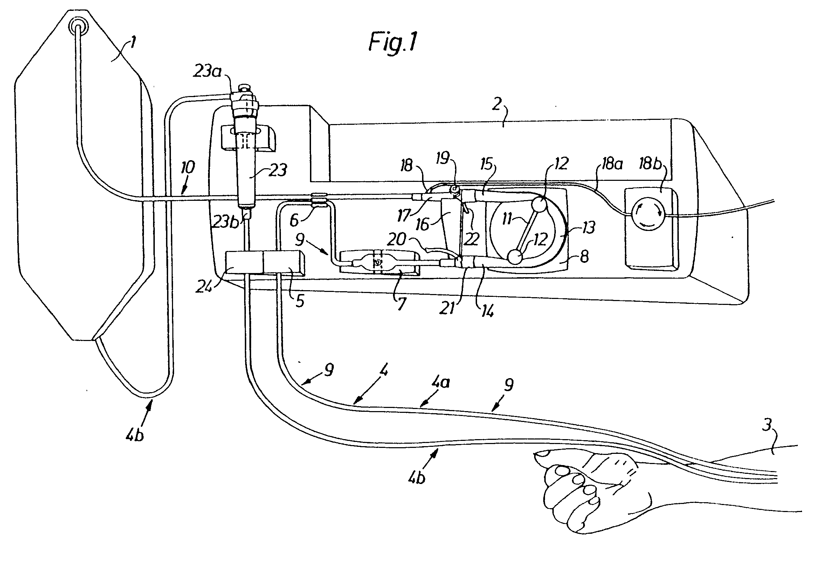

Fig. 1 thus shows a dialyser 1, a control unit 2 and a patient 3 coupled together by means of a tube set designated as a whole by 4. This tube set consists of an arterial part 4a and a venous part 4b. The arterial part 4a is connected by means of an injection needle, not shown, to one of the arteries of the patient 3 and passes through a shut-off clip 5, a holder 6 and an arterial pressure monitor 7 to a pump 8. The part extending to the suction side of the pump constitutes the inlet part of the arterial sets and is designated as a whole by 9. The part between the delivery side of the pump and the dialyser 1 constitutes the outlet part of the arterial set and is designated as a whole by 10.

Since the invention resides first and foremost in the tube set shown, the control unit 2 is shown only schematically without the instruments and many other components which are normally included in such a control unit. The pump 8, in additino to the rotor 11 with its two rollers 12 firmly installed in the control unit, comprises a pump segment 13 incorporated in the tube set. The inlet end 14 and the outlet end 15 of this pump segment 13 are rigidly connected with one another via a spacer 16. This spacer comprises between the outlet end 15 of the pump segment and the outlet part 10 of the tube set a pipe fitting 17 connecting them. This pipe fitting 17 in turn comprises an inlet 18 for the input of a reagent, e.g. heparin, and a port 19 for sampling arranged upstream of this inlet. The distances between these components in respect of the outlet end 15 of the pump segment are chosen so that sampling can take place without any danger of interference by the reagent added through back suction from the pump segment 13. A source of heparin or the like is connected to the port or inlet nozzle 18 by a tube 18a via a pump 18b.

Between the inlet part 9 of the tube set and the inlet end 14 of the pump segment the spacer 16 is provided with a pipe fitting 21 connecting them. This pipe fitting too comprises a port, in this case in the form of a closed tube end 20 for the input of a liquid e.g. priming liquid, with the help of the pump 8. Finally the spacer comprises a stay 22 intended to support it against the front of the control unit or any other component firmly attached to the pump 8.

From the dialyser 1 the venous part 4b of the tube set passes via a drip chamber 23 with an inlet 23a and an outlet 23b and via a shut-off clip 24 back to the patient 3.

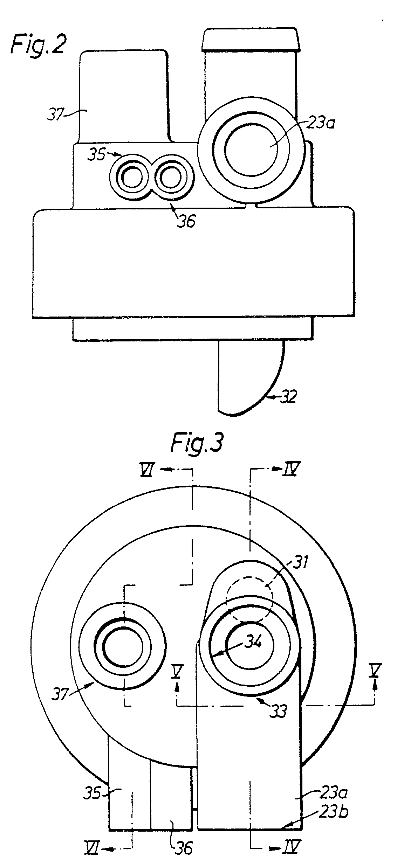

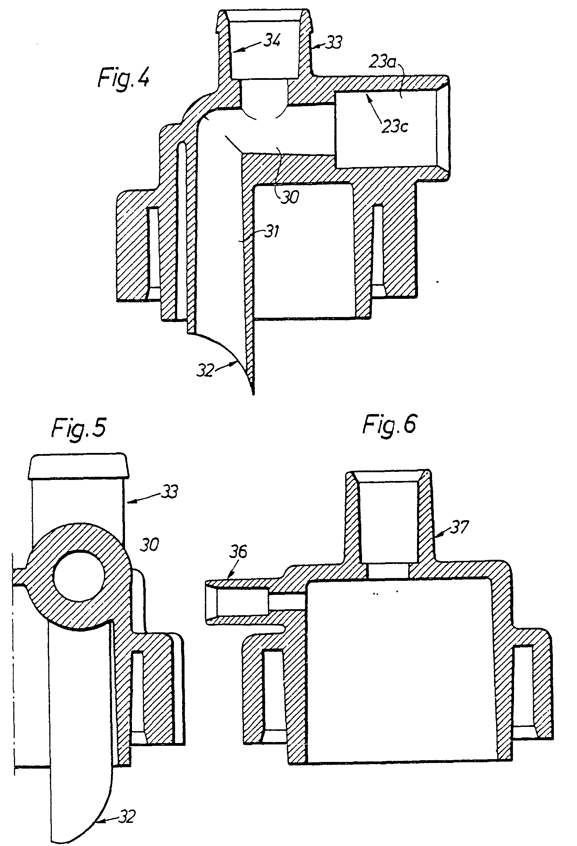

Fig. 2—6 show the top part of the drip chamber 23 in more detail. Thus the inlet 23a is arranged from the side, that is to say horizontally in relation to the drip chamber intended for vertical installation. The inlet 23a is provided with a seat 23c for the tube part arranged between the dialyser and the drip chamber. From the inlet 23a extends a horizontal duct 30 which is directed into a vertical duct 31, the lower part 32 of which has been given a soft rounding so as to bring about a suitable drip formation. Straight before the horizontal duct 30 is arranged a sampling port 33. This port 33 is arranged therefore before the point where the liquid flow is diverted from the duct 30 to the vertical duct 31. The seat 34 in the port 33 is intended to hold a rubber stopper or the like through which can be inserted a syringe. To allow the concentrating of further functions on the drip chamber, the lidlike part is provided with further ports 35, 36 and 37. The port 35, for example, may be used for level control which may be carried out through the feed or withdrawal of air with the help of an ordinary syringe.

The port 36 may be connected to a pressure gauge for the monitoring of the pressure in the venous line 4b. Finally the port 37 can be intended for the infusion of a dilution liquid, e.g. makeup liquid in haemofiltration. Thus all the functions which are customary in a venous set have been concentrated on the drip chamber and in particular on its lidlike top part.

Naturally, the invention is not limited simply to the preferred embodiment described above, but can be varied within the scope of claim 1. The different ports mentioned above, for example, may be arranged so as to fulfil functions other than those mentioned by way of example. Furthermore the tube set can be provided, of course, with further components so as to perform other functions.

## Claims

1. A tube set intended for extracorporeal treatment of blood and similar perishable liquids, e.g. in conjunction with dialysis, comprising at least one drip chamber (23) with an inlet (23a) and an outlet (23b) arranged at an angle in relation to one another, characterized by a sampling port (33) arranged in the drip chamber (23) in connection with the inlet (23a) upstream of a point where a duct extending from the inlet (23a) is diverted downwards into the drip chamber (23).

2. A tube set in accordance with claim 1, the drip chamber (23) having an elongate shape with the outlet (23b) at its lower end and arranged in its longitudinal direction, characterized in that the inlet (23a) is arranged from the side in a lidlike part.

3. A tube set in accordance with any one of the preceding claims, characterized in that the drip chamber (23) comprises moreover, one or more ports (35, 36, 37) for the withdrawal and/or feed of a material, e.g. a liquid or air.

4. A tube set in accordance with claim 3, characterized in that one or more of the said ports (35, 36, 37) are arranged from the side in a lidlike part.

5. A tube set in accordance with any one of the preceding claims intended to constitute a venous set (4b) in dialysis or similar treatment, characterized in that all functions are concentrated on the drip chamber (23) as a result of which the tube parts (4b, 4b) included are completely unbroken between the dialyser (1) or corresponding treatment arrangement and the drip chamber (23) as well as between the latter and the patient.

## Patentansprüche

1. Schlauchsystem für die extrakorporale Behandlung von Blut und ähnlichen leichtverderblichen Flüssigkeiten, zB in Verbindung mit einer Dialyse, wobei das Schlauchsystem mindestens eine Tropfkammer (23) mit einem Einlass (23a) und einem Auslass (23b) aufweist, welche in einem Winkel relative zueinander angeordnet sind, gekennzeichnet, durch einen Probeentnahmeanschluss (33), welcher in der Tropfkammer in Verbindung mit dem Einlass (23a) stromaufwärts von einem Punkt angeordnet ist, wo ein Leitungskanal, der sich von dem Einlass (23a) aus erstreckt, nach unten in die Tropfkammer (23) abzweigt.

2. Schlauchsystem nach Anspruch 1, wobei die Tropfkammer (23) eine längliche Form hat mit einem Auslass (23b) in ihrem unteren Ende und angeordnet in ihrer Längsrichtung, dadurch gekennzeichnet, dass der Einlass (23a) von der Seite her in einem deckelartigen Teil angeordnet ist.

3. Schlauchsystem nach irgendeinem der vorstehenden Ansprüche, dadurch gekennzeichnet, dass die Tropfkammer (23) weiterhin einen oder mehrere Anschlüsse (35, 36, 37) für die Entnahme und/oder Zufuhr eines Materials, zB einer Flüssigkeit oder Luft, aufweist.

4. Schlauchsystem nach Ansprüch 3, dadurch gekennzeichnet dass einer oder mehrere der Anschlüsse (35, 36, 37) von der Seite her in einem deckelartigen Teil angeordnet sind.

5. Schlauchsystem nach irgendeinem der vorstehenden Ansprüche, welches vorgesehen ist, um ein venöses System (4b) bei der Dialyse oder einer ähnlichen Behandlung zu bilden, dadurch gekennzeichnet, dass alle Funktionen an der Tropfkammer (23) konzentriert sind, so dass als Folge hiervon die enthaltenden Schlauchteile (4b, 4b) zwischen dem Dialysegerät (1) oder einer entsprechenden Behandlungsanordnung und der Tropfkammer (23) ebenso wie zwischen der letzteren und dem Patienten vollständig ohne Unterbrechung sind.

**Revendications**

1. Tubulure destinée au traitement extracorporel du sang et de liquides pétrissables analogues, par exemple en relation avec la dialyse, comprenant au moins une chambre d'écoulement (23) présentant un orifice d'entrée (23a) et un orifice de sortie (23b) disposés angulairement l'un par rapport à l'autre, caractérisée par un orifice de prélèvement (33) disposé dans la chambre d'écoulement en liason avec l'orifice d'entrée (23a) en amont d'un point où un conduit partant de l'orifice d'entrée (23a) est dévie vers le bas dans la chambre d'écoulement.

2. Tubulure suivant la revendication 1, la chambre d'écoulement (23) ayant une forme allongée avec l'orifice de sortie (23b) à son extrémité inférieure et disposé dans son sens longitudinal, caractérisée en ce que l'orifice d'entrée (23a) est disposé latéralement dans une partie formant couvercle.

3. Tubulure suivant l'une quelconque des revendications précédentes, caractérisée en ce que la chambre d'écoulement (23) comprend en outre un ou plusieurs orifices (35, 36, 37) pour le retrait et/ou l'introduction d'un matériau tel qu'un liquide où de l'air.

4. Tubulure suivant la revendication 3, caractérisée en ce qu'un ou plusieurs des orifices précités (35, 36, 37) sont disposés latéralement dans une partie formant couvercle.

5. Tubulure suivant l'une quelconque des revendications précédentes, destinée à constituer une ligne veineuse (4b) dans un traitement de dialyse ou analogue, caractérisée en ce que toutes les fonctions son concentrées sur la chambre d'écoulement (23), d'où il résulte que les parties (4b, 4b) de la tubulure incluses entre le dialyseur (1) ou un appareil de traitement correspondant et la chambre d'écoulement (23) ainsi qu'entre cette dernière et le patient sont complètement exemptes de solution de continuité.

Fig.1

1

*Fig.2*

37

35

23a

36

32

*Fig.3*

VI      IV

31

34

37

33

35

23a

23b

VI      36      IV

Fig. 4

Fig. 5

Fig. 6